(19) Europäisches Patentamt / European Patent Office / Office européen des brevets

(11) **EP 4 300 371 A1**

(12) **EUROPEAN PATENT APPLICATION**

(43) Date of publication:
**03.01.2024 Bulletin 2024/01**

(21) Application number: **22182114.3**

(22) Date of filing: **30.06.2022**

(51) International Patent Classification (IPC):
***G06N 3/08*** *(2023.01)*

(52) Cooperative Patent Classification (CPC):
**G06N 3/084**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **OSADA, Genki**
  **Eindhoven (NL)**
- **AHSAN, Budrul**
  **Eindhoven (NL)**

(74) Representative: **Philips Intellectual Property & Standards**
**High Tech Campus 52**
**5656 AG Eindhoven (NL)**

(54) **CORRECTING MACHINE-LEARNING MODEL TRAINING DATA**

(57) Proposed concepts thus aim to provide schemes, solutions, concepts, designs, methods and systems pertaining to improving (i.e. increasing accuracy and/or reliability) machine-learning models by correcting data used to train such models. In particular, a timestamp of training data describing an event is modified according to a time-shift function and a predetermined time uncertainty range. In this way, an uncertainty/inaccuracy of the recording of the timestamp may be compensated for, such that a quality of the training data may be improved.

200

| Obtain time-series data and event data | 210 |
| Correct the event data | 100 |
| Train a status prediction model using the corrected event data and the status data | 220 |

FIG. 2

EP 4 300 371 A1

**Description**

FIELD OF THE INVENTION

The present invention relates to the field of training machine-learning models, and in particular to the field of correcting data used for training machine-learning models.

BACKGROUND OF THE INVENTION

Event data, being data related to an occurrence of an event, is often recorded manually. As a result, a timestamp of the event described by the event data is highly liable to being inaccurate due to recording/human error, and/or because the inaccuracy of a clock used to reference the time of the event.

In many fields, event data is used to train machine-learning (i.e. artificial intelligence) models. As the quality of a machine-learning model heavily depends on the quality of the data that is used to train them, inaccuracies associated with recorded event data are problematic.

By way of specific example, in the field of medicine, machine-learning models can be used to predict the future condition of a subject. This may be particularly beneficial during surgical operation, where such predictions may be important for subject safety. However, as it is not uncommon for the timestamp for event data (i.e. a drug administration event, a subject repositioning event, etc.) to be recorded incorrectly, the accuracy and reliability of such machine learning models may be reduced. Ultimately, this may threatening the safety of subjects.

Thus, there exists a present need for a means to mitigate the impact of such inaccuracies in event data used to train machine-learning models.

SUMMARY OF THE INVENTION

The invention is defined by the claims.

According to examples in accordance with an aspect of the invention, there is provided a method for correcting machine-learning model training data, the method comprising:

   obtaining training data comprising a timestamp value describing a timing of an event occurrence; and modifying the timestamp value of the obtained training data according to a time-shift function configured to adjust the timestamp value based on a predetermined time uncertainty range.

Proposed concepts thus aim to provide schemes, solutions, concepts, designs, methods and systems pertaining to improving (i.e. increasing accuracy and/or reliability) machine-learning models by correcting data used to train such models. In particular, a timestamp of training data describing an event is modified according to a time-shift function and a predetermined time uncertainty range. In this way, an uncertainty/inaccuracy of the recording of the timestamp may be compensated for, such that a quality of the training data may be improved.

It is known that data describing events often have unreliable (i.e. inaccurate, imprecise, etc.) timestamps. This is due to timestamps in such cases typically being recorded by individuals, who are liable to error from a variety of sources. As such, when said unreliable data is used to train machine-learning models, the quality of the model may be compromised.

Embodiments of the invention thus aim to overcome such problems by correcting/modifying data used for training machine-learning models. This is achieved by adjusting the original timestamp value by a time-shift function, which accounts for an uncertainty of the timestamp value. In some embodiments, the timestamp value may be shifted by a random amount within a predetermined time uncertainty range. When this modification of the timestamp value is applied to training data describing timings of many event occurrences, the impact of any errors in individual timestamp values for training a machine-learning model may be suppressed.

In other words, by modifying the timestamp value in this way, a resolution of the timestamp values of all of the event occurrences may be reduced. This drop in resolution may correspond to an uncertainty, thus negating the impact of errors in recording the timestamp.

In many cases, events are often the trigger for subsequent changes in systems. By way of example, a drug administration event will usually trigger changes in a physiological state of a subject. An accurate understanding the timing of such an event is critical for understanding the causal relationship between an event occurrence, and an impact of the event. However, in many environments, the accurate recording of the timing of such events may not be a priority, or may not be possible. Thus, when data gathered around such events is used to train a machine-learning model, the machine-learning model may have an incorrect understanding of the causal relationship between events and subsequent system changes. Therefore, by accounting for these inconsistencies in recorded timestamps via a time-shift function and a predetermined time uncertainty range, machine-learning model training data may be improved.

Therefore, in the present invention, the timestamp value describing a timing of an event occurrence is modified/shifted/altered - reducing an effective precision of the timestamp, but improving accuracy of the timestamp.

In some embodiments, the predetermined time uncertainty range may be indicative of a predicted difference between the timestamp value and an actual timing of the event occurrence.

Preferably, the modification of the timestamp value should not be greater than a difference between the recorded timing of the event, and a ground-truth timing of the event. This ensures that an effective precision of the timestamp is not reduced any more than is necessary to improve the accuracy of the timestamp.

In some embodiments, the predetermined time uncertainty range may be based on an event type corresponding to the event occurrence.

Indeed, an uncertainty associated with the timestamp is often heavily linked with the type of event to which the timestamp describes the occurrence. For example, timestamps of events where it is difficult for the recorder to discern the precise timing of the event may have a higher level of uncertainty. Furthermore, the uncertainty may be higher for events which inherently necessitate the recording of the timestamp retrospectively (i.e. when the recorder is involved in the actuating of the event, or where the recorder cannot have any recording apparatus to hand).

Put another way, information related to the event type may be leveraged in order to determine a likely error in the recording of the timestamp. Thus, exploitation of this information may result in a more appropriate predetermined time uncertainty range for the modification of the timestamp value to be based upon.

In some embodiments, the time-shift function may be configured to adjust the timestamp value based on the predetermined time uncertainty range and a probability distribution algorithm.

Accordingly, a modification of the timestamp value may be appropriately performed. In this case, when the modification is applied to many timestamp values, an average accuracy may be further improved.

In some embodiments, the probability distribution algorithm may follow a uniform distribution. In other embodiments, the probability distribution algorithm may follow a normal distribution. In yet further embodiments, the probability distribution algorithm may follow an asymmetric probability distribution, and preferably a lognormal distribution.

Different types of probability distribution algorithms may be more appropriate for different types of use cases. This may depend on a type of event described, or a preference of a user wanting to correct the training data.

According to further aspects of the invention, there is provided a method of generating a status prediction model adapted to output a status prediction indicative of a future physiological state of a subject, the method comprising: obtaining time-series data comprising status data describing at least one physiological characteristic, and event data comprising a timestamp value describing a timing of an event occurrence; correcting the event data according to a method for correcting machine-learning model training data; and training a status prediction model using a training algorithm configured to receive an array of training inputs and known outputs, wherein the training inputs comprise the corrected event data and the status data, and the known outputs comprise the status data.

According to other aspects of the invention, there is provided a method of generating a status prediction model adapted to output a status prediction indicative of a future physiological state of a subject, the method comprising: obtaining time-series data comprising status data describing at least one physiological characteristic, and event data comprising a timestamp value describing a timing of an event occurrence; correcting the event data by modifying at least one of the timestamp values of the event data according to a time-shift function configured to adjust the timestamp value based on a predetermined time uncertainty range; and training a status prediction model using a training algorithm configured to receive an array of training inputs and known outputs, wherein the training inputs comprise the corrected event data and the status data, and the known outputs comprise the status data.

Thus, the above-described method of correcting/modifying training data may be leveraged to train a status prediction machine-learning model, such that the status prediction model may output a more accurate and/or reliable predictions related to a future physiological state of a subject.

Indeed, a future status of a subject is heavily dependent upon events that occur to subjects (i.e. a drug administration event, a treatment event, a repositioning event), as well as a present status of the subject. The status of a subject is generally recorded by sensors (i.e. a vital sign monitor), which inherently means that timestamps are accurate. However, event data is commonly recorded by caregivers, who may record a time of the event retrospectively, and who are in a high-pressure environment where errors may be common. Thus, timestamp values of events related to the subject may be highly inaccurate.

Thus, it is usually the case that status prediction models are trained on datasets which contain many errors, leading to inaccurate and unreliable output predictions. Embodiments of the present invention aim to mitigate this problem by modifying timestamps according to a time-shift function configured to adjust the timestamp value based on a predetermined time uncertainty range. Accordingly, an improved (i.e. more accurate and reliable) status prediction model may be trained.

In some embodiments, the status data may comprise vital sign data. The status data may further comprise at least one of a heart rate, a blood pressure, and an oxygen saturation level.

Such data may be acquired automatically by sensors attached to the subject. Thus, a timestamp of the status data may be considered to be close to the ground-truth timing.

In some embodiments, the event data may comprise intervention information describing a subject treatment. The event data may comprise at least one of a drug administration event, a movement event, and a treatment event.

Such data may correspond to timestamp values that are inaccurate due to human error. However, such described events may also have a significant impact on a status of the subject. Therefore, an accurate timestamp value is required to properly assess the link between the event and the status of the subject, as well as predict future statuses.

In some embodiments, the training algorithm is a stochastic gradient descent algorithm.

According to yet further aspects of the invention, there is provided a method of generating a status prediction indicative of a future physiological state of a subject, the method comprising: generating a status prediction model according to a method of generating a status prediction model adapted to output a status prediction indicative of a future physiological state of a subject; obtaining time-series data associated with the subject, the time-series data comprising status data describing at least one physiological characteristic of the subject, and event data comprising a timestamp value describing a timing of an event occurrence corresponding to the subject; acquiring the subject status prediction based on inputting the time-series data to the generated status prediction model.

Accordingly, by generating a status prediction model using corrected training data as described above, an improved (i.e. more accurate, precise and reliable) subject status prediction may be acquired. This may have a significant positive impact on subject outcomes, as predicting a future status is key for determining appropriate steps in subject care.

According to further aspects of the invention, there is provided a computer program comprising computer program code means adapted, when said computer program is run on a computer, to implement a method for correcting machine-learning model training data, generating a status prediction model adapted to output a status prediction indicative of a future physiological state of a subject, and generating a status prediction indicative of a future physiological state of a subject.

According to additional aspects of the invention, there is provided a system for correcting machine-learning model training data, the system comprising: an interface configured to obtain training data comprising a timestamp value describing a timing of an event occurrence; and a data manipulation unit configured to modify the timestamp value of the obtained training data according to a time-shift function configured to adjust the timestamp value based on a predetermined time uncertainty range. These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 presents a flow diagram of a method for correcting machine-learning model training data according to an embodiment of the invention;

Figure 2 presents a flow diagram of a method for generating a status prediction model adapted to output a status prediction indicative of a future physiological state of a subject according to a further embodiment of the invention;

Figure 3 presents a flow diagram of a method for generating a status prediction indicative of a future physiological state of a subject according to another embodiment;

Figures 4A-4C depict an illustration of a correction of timestamps of event data in time-series data according to an aspect of an embodiment of the invention;

Figure 5 presents a simplified block diagram of a system for correcting machine-learning model training data according to an embodiment; and

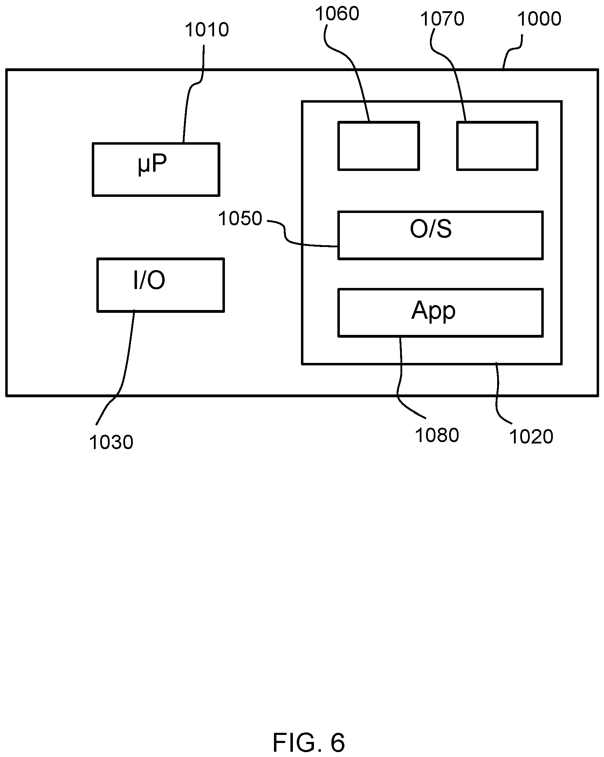

Figure 6 is a simplified block diagram of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for enabling the improvement (i.e. increasing accuracy and/or reliability) of machine-learning models by correcting data used to train such models. In particular, a timestamp of training data describing an event is modified according to a time-shift function and a predetermined time uncertainty range. In this way, an uncertainty/inaccuracy of the recording of the timestamp may be compensated for, such that a quality of the training data may be improved.

Indeed, it has been realized that an accuracy of timestamp values corresponding to (critical) event data may be increased by individually adjusting timestamp values randomly within a range of values. For example, while it may not be accurate that an event occurred at a specific time, it may be accurate that an event occurred within a 10 minute period. Thus, the invention may result in an effective loss of precision for the timestamp values, but an improved accuracy. In other words, a resolution of the timestamp value is decreased in order to ensure accuracy.

Accordingly, this invention for correcting timestamps may be used in a variety of fields for improving machine-learning models. The invention may be applied to any training dataset that utilizes timestamps, wherein the timestamps may be considered inaccurate, or it is uncertain as to the accuracy of the timestamps. This may be particularly beneficial for the medical domain, and more specifically for predicting a subject's future physiological status based on current/past status data, and event data. Indeed, timestamps of the event data may be highly inaccurate in such a field, and therefore correction of training data sets may lead to improved (i.e. accurate and reliable) machine-learning models.

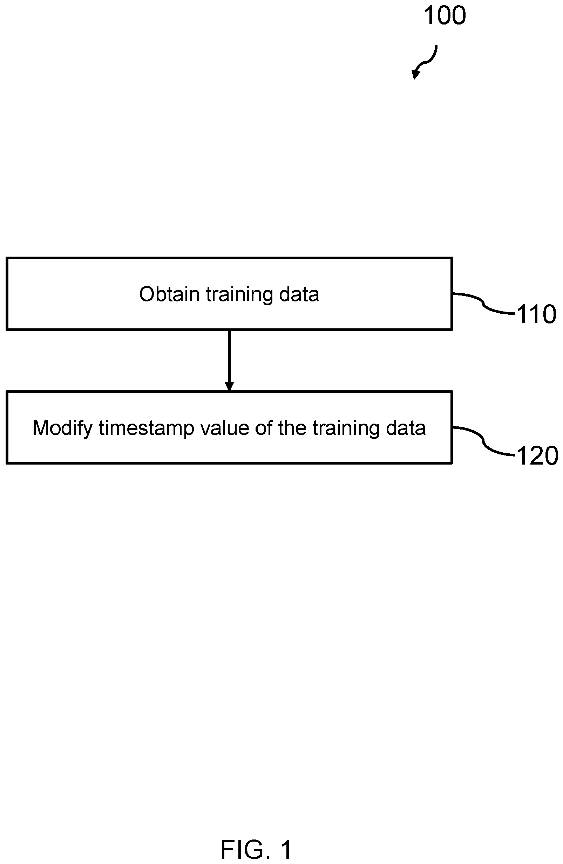

Turning now to Figure 1, there is depicted a flow diagram of a method 100 for correcting/modifying/changing (i.e. improving the accuracy) machine-learning model training data according to an embodiment of the invention. Put briefly, the method 100 may receive data that is to be used to train a machine learning model, and alter timestamps of the data such that the timestamps are accurate. This is achieved by reducing the overall precision of the timestamps of the data (i.e. reducing a resolution of the timestamp).

Specifically, at step 110, training data comprising a timestamp value describing a timing of an event occurrence is obtained. Put another way, a timestamp value (i.e. a date-time value) which indicates when an event described by each training data point, is obtained/received.

Indeed, the (recorded) timestamp value may have been recorded by a human (i.e. not automatically), and therefore may have inaccuracies. In other embodiments, the timestamp value may have inaccuracies due to differences in clocks of sensors used to detect the occurrence of an event. Some of the timestamp values of the training data may be accurate (i.e. be equivalent to the ground-truth). However, overall the timestamp values of the training data have an associated uncertainty as to the accuracy of the value.

The (plurality of) training data may be obtained from a database, or may be received as it is recorded. Alternatively, the training data may be retrieved from a corpus of information gathered from a variety of sources.

At step 120, the timestamp value of the obtained training data is modified according to a time-shift function configured to adjust the timestamp value based on a predetermined time uncertainty range.

In other words, as the timestamp values have an associated overall level of uncertainty/inaccuracy (i.e. due to sources of inaccurate recording), they may be altered/changed/modified according to the overall level of uncertainty, in an attempt to compensate for the overall level of uncertainty/inaccuracy. For example, it may be known that the event may have occurred within 10 minutes of the timestamp value (i.e. as it was a guess by the recorder). In this case, the timestamp value is modified with this inaccuracy in mind.

The time-shift function is a function that takes the (recorded) timestamp value and a predetermined time uncertainty range as input, and outputs a modified/altered timestamp value. This modification may be deterministic (i.e. be fixed based on the timestamp value), or may be probabilistic (i.e. be determined based on randomness within a variance range). In certain embodiments, the training data with corresponding modified timestamp values may then be used to train a machine-learning model.

In some embodiments, the time-shift function may configured to adjust/modify the timestamp value based on the predetermined time uncertainty range and a probability distribution algorithm. Thus, the (plurality) of timestamp values may be changed according to an algorithm configured to distribute the timestamp values along a predetermined time uncertainty range. By way of example, one timestamp value may be moved forward by a few minutes, another timestamp value may be moved backward by a few minutes, and another timestamp value may not be changed at all.

More specifically, the probability distribution algorithm may follow one of a uniform distribution, a normal distribution, an asymmetric probability distribution, and a lognormal distribution. Indeed, the probability distribution algorithm may follow any distribution suitable for altering timestamps randomly within a range known to the skilled person. In each case, the distribution may take a value based on the predetermined time uncertainty range as input, as well as the timestamp value, to output a modified/corrected timestamp value.

Moving on, the predetermined time uncertainty range may be a fixed value, set according to user preferences. Alternatively, the predetermined time uncertainty range may be automatically set according to information about the timestamps and/or events.

Furthermore, the predetermined time uncertainty/inaccuracy range may be indicative of a predicted difference between the timestamp value and an actual timing of the event occurrence. Indeed, the predetermined time uncertainty range may be thought of as a value range of possible actual timings that the event described by the timestamp value had occurred.

In some embodiments, the predetermined time uncertainty range may be based on an event type corresponding to the event occurrence. Indeed, the event type may be a factor in the level of uncertainty associated with the timestamp. For example, when the event type necessarily means that the person recording the event cannot record the timestamp as the event occurs, this may indicate a higher level of uncertainty than the contrary.

While the invention (of correcting timestamp values of training data) described in relation to Figure 1 will now be described in use for generating a status prediction model, it should be understood that it may be applied to a wide variety of fields. Indeed, the invention may be applied to any data set to be used for training a machine-learning model, having timestamps that may be considered inaccurate.

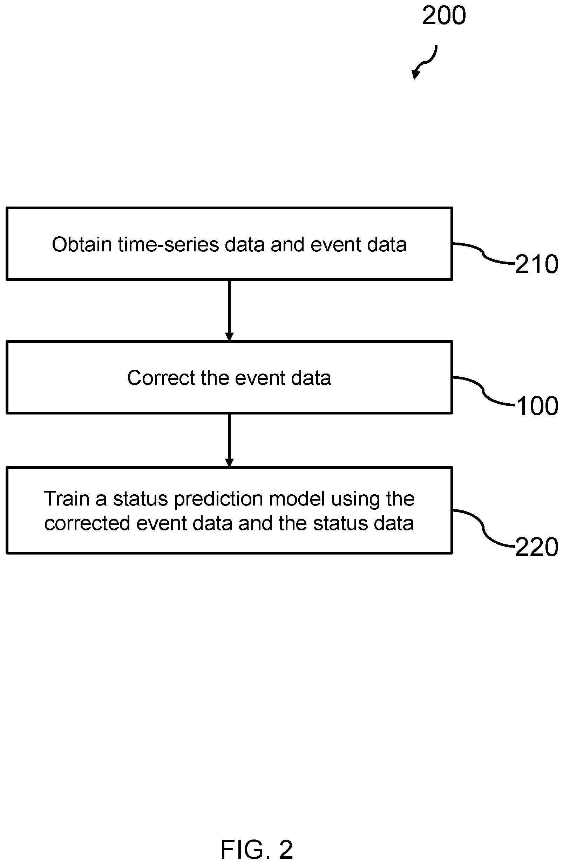

Figure 2 is a flow diagram of a method 200 for generating a status prediction model adapted to output a status prediction indicative of a future physiological state of a subject according to an embodiment of the invention. In other words, the method 200 provides a machine-learning model for generating predictions related to a status of a subject responsive to an input of time-series data of the subject.

At step 210, time-series (training) data is obtained. The time-series data comprises status data describing at least one physiological characteristic (i.e. a characteristic of a subject that indicates a biological attribute of a subject), and event data (i.e. data describing a change in circumstance of a subject) comprising a timestamp value describing a timing of an event occurrence.

Put another way, time-series data describing a subject over time is obtained/received. This data may be in real-time, obtaining the data directly from sensors/directly as it is recorded, or may be received from a centralized storage. The time-series data may describe a status and event of one subject over time, or may correspond to a plurality of different subjects.

The status data may comprise vital sign data, including at least one of a heart rate, a blood pressure, and an oxygen saturation level. It should be noted that the skilled person would understand that the status data may include any physiological (i.e. biological) data which may be useful for determining the state of a subject.

The event data may comprise intervention information describing a subject treatment, including at least one of a drug administration event, a movement event, and a treatment event. It should be noted that the skilled person would understand that the event data may include any data relating to a change of circumstances of a subject, which may have an impact on a status of the subject.

The method 200 then moves to step 100, where the event data is corrected. This may be achieved according to the method 100 described above in reference to Figure 1. In any case, at least one of the timestamp values of the event data is modified according to a time-shift function configured to adjust the timestamp value based on a predetermined time uncertainty range. In this way, inaccuracies in timestamps of the event data may be accounted for, improving the quality of the event data.

At step 220, a status prediction model (i.e. a neural network based machine learning model or artificial intelligence model) is trained using a training algorithm configured to receive an array of training inputs and known outputs, wherein the training inputs comprise the corrected event data and the status data, and the known outputs comprise the status data.

In other words, the status prediction machine-learning model is trained based on the status data and the corrected event data. The training may be by any method known in the art. For example, the status prediction model may be trained using a stochastic gradient descent algorithm.

By way of explanation, the structure of an artificial neural network (NN) based machine-learning algorithm is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs), recurrent neural networks (RNNs) and graph neural networks (GNNs).

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the status prediction machine-learning model used in method 200 correspond to example status data and event data. The training output data entries correspond to status data describing a status of a subject responsive to a previous status and a previous event. That is, the machine learning algorithm is trained using a training algorithm configured to receive an array of training inputs and respective known outputs, wherein a training input comprises status data and event data, and respective known output comprises subsequent status data. In this way, the machine learning algorithm is trained to output a status prediction indicative of a future physiological state of a subject.

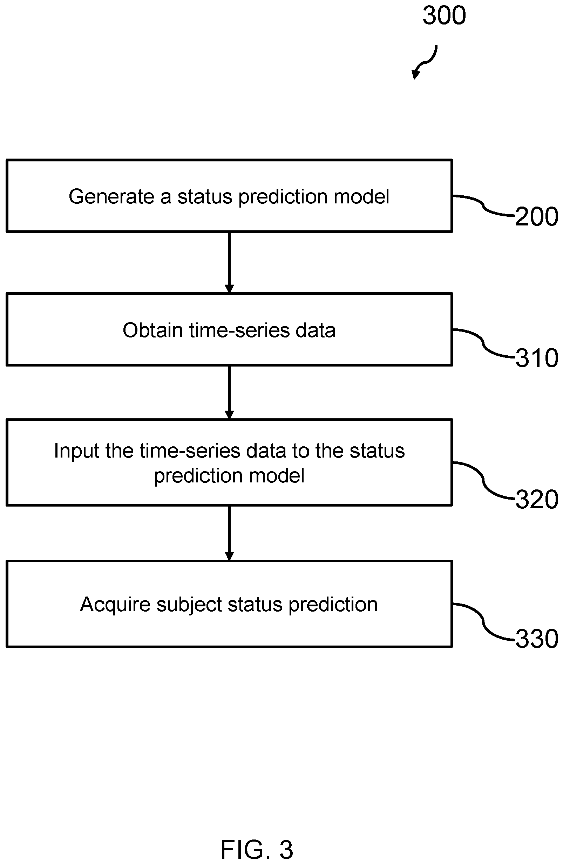

Figure 3 presents a flow diagram of a method 300 for generating a status prediction indicative of a future physiological state of a subject. Indeed, this may be particularly useful in a medical environment, where prediction as to the future state of the subject can lead to better decisions of actions to take.

Firstly, a status prediction model is generated. This is achieved according to an embodiment of the method 200 described in relation to Figure 2. As a result, a status prediction machine learning model is obtained that may generate a status prediction indicative of a future physiological state of a subject, based on time-series data of the subject.

The method 300 then moves on to step 310, where time-series data associated with the subject is obtained. The time-series data comprises status data describing at least one physiological characteristic of the subject, and event data comprising a timestamp value describing a timing of an event occurrence corresponding to the subject. This time-series data may be similar to the data used to train the status prediction model, but is related to the subject.

The event data may describe an event that the subject has experience, or may be a hypothetical event that is used to determine an anticipated change in the status of the subject should the event to occur.

At step 320, a subject status prediction is acquired, the subject status prediction based on inputting the time-series data to the generated status prediction model. Such a subject status prediction may be used by caregivers to choose appropriate actions/treatments, improving subject outcomes.

By way of an illustrative example, an embodiment of the invention for predicting a future subject status will now be described. More specifically, biometric (i.e. subject status) monitoring during clinical anaesthesia is essential for subject safety. Existing systems and methods may detect the occurrence of abnormalities such as hypotension and bradycardia, and alert a caregiver/anaesthesiologist with an alarm. However, by the time the alarm is triggered, the anomalous situation has usually already begun.

Enabling an advanced/early alert for caregivers and anaesthesiologists as to the subject's status/condition will be may thus be incredibly useful. Even if the alert is only a matter of minutes early, appropriate actions and measures may be taken to improve subject outcomes. Thus, predicting a future subject status is very useful in practice.

In order to predict a subject's future status, the predictive capabilities of machine-learning models may be employed. Input data to the machine-learning model consists of current status data (i.e. vital signs, such as blood pressure and heart rate), and event data (i.e.

records of intervention, such as drug administration and patient repositioning). Taking the input data, the machine-learning model outputs a status subject prediction. For example, the subject status prediction may related to the subject's blood pressure in the future.

However, a problem for training the machine-learning model is the inaccuracy of timestamp values associated with the event data. Usually, subject status data is automatically collected from sensors and recorded in real-time, so the timestamp value associated with such data may be substantially accurate. On the contrary, commonly event data is manually entered into the system after the procedure/event. Caregivers often perform medical procedures first, and enter the event data (including the corresponding timestamp) after the event trigger has been resolved. Therefore, the timestamp value for event data naturally tends to deviate from the actual, ground-truth timestamp value.

Moreover, compared to vital sign data, the volume of event data available is usually quite small. However, event data (in particular data related to drug administration), has a very large impact upon a future status of the subject, and a prediction thereof. This is because most of those events are performed exactly for the purpose of controlling the status of the subject (i.e. subject vital signs including blood pressure and heart rate). Thus, timestamp value inaccuracy in the rare/sparse but highly influential event data used to train a machine-learning model degrades the predictive performance of the machine-learning model. Embodiments of the invention aim to overcome such inaccuracy, while utilizing informative event data.

In order to provide a solution to the above problems, embodiments of the invention include the following:

(i) Input of time-series data. Each unit of the time-series data has a corresponding timestamp value. The time-series data may consists of several columns of subject status data (i.e. vital signs), and event data (i.e. intervention records).

(ii) Machine-learning model. The machine-learning model makes output predictions of a future status of the subject based on the input of time-series data. The machine-learning model is trained by a training algorithm.

(iii) The training algorithm. For example, the training algorithm may be a stochastic gradient descent (SGD), which is used to train the machine learning model. The training algorithm may read a sample of the time-series data at a time (which is called a mini-batch, a small amount of data used in a single training iteration) and update (i.e. trains) parameters of the machine-learning model based on the time-series data. In order to train the machine-learning algorithm effectively, this operation is repeated many times, such as tens of thousands to millions of times.

(iv) A data manipulation unit. The data manipulation corrects/modifies/changes the timestamp corresponding to the event data in a certain given range. The data manipulation unit may be used every time the training algorithm is used.

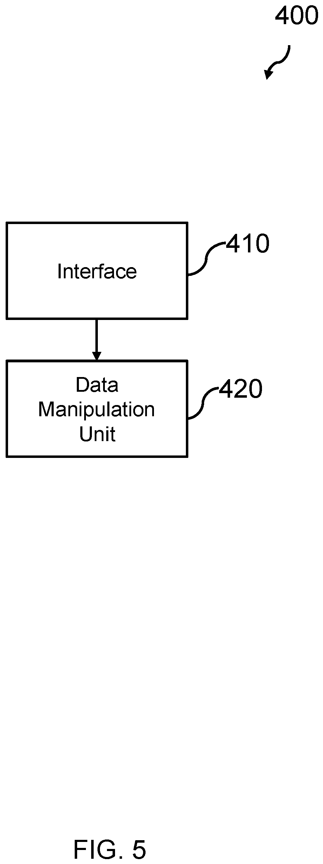

An example of the data manipulation unit function will be discussed in more detail in relation to Figures 5A-5C, which depict an illustration of the modification of timestamps of event data in time-series data according to an aspect of an embodiment of the invention.

For example, an event occurrence with a timestamp value being equal to 10:03 may have occurred a little earlier than that, such as perhaps at 10:01. In this sense, the timestamp value is incorrect. However, if the timestamp value instead reflects that the event occurs between 10:00 and 10:05, then it is no longer wrong.

Indeed, this is presented in the second and third column of Figure 5A-5C, which record the occurrence of events Med_A and Med_B. These events have corresponding timestamp values of 27:00:00 and 34:00:00, respectively. A timeshift function is then applied in Figure 5B, which moves Med_A to have a timestamp value of 24:00:00 and moves Med_B to have a timestamp value of 36:00:00. Indeed, Figure 5C presents the range of values that the timestamp value may have had.

Put another way, embodiments of the invention intentionally reduces the resolution of the timestamp value of the event occurrence. This makes it possible to overcome the inaccuracy problem and allows the use of incorrect event data while still effectively training the machine-learning model.

This effective resolution reduction may be achieved by randomly shifting the timestamp value of the event data within a certain specified width (i.e. a predetermined time uncertainty range).

By way of example, suppose that the timestamp of administration of drug A is recorded as 10:03. By modifying the timestamp value, the timestamp value may be between 10:07 and 9.59. Indeed, this may be written as:

$$t\_new = t + ep$$

where t_new is the modified timestamp, ep is a value sampled at random, and t is the recorded/original timestamp value.

If it is desirable to shift the timestamp value evenly within 3 minutes before and after the original timestamp value, a uniform distribution may be used:

$$ep \sim U(-180, 180)$$

Alternatively, a normal distribution may be used:

$$ep \sim 180 * N(0, 1)$$

In many cases, we may know that a timestamp for event data for some events tends to lag the real-time of the timestamp, and is rarely earlier. In such cases, an asymmetric probability distribution may be used, such as lognormal distribution:

$$ep \sim - 180 * \log N(0, 1)$$

To reiterate, the training of the machine learning model may be performed by repeating the process of updating parameter values of the machine learning model many times. The parameter update may be performed based on the part of the time-series data, called a mini-batch. The time-shift function is applied to each mini-batch. Further, the time-shift function is applied separately for each timestamp of different events.

Accordingly, resolution reduction of the timestamp by random shifting may address the issue of event data having inaccurate timestamps. The invention may be applied to sporadic event data in many different fields, to effectively train machine-learning models.

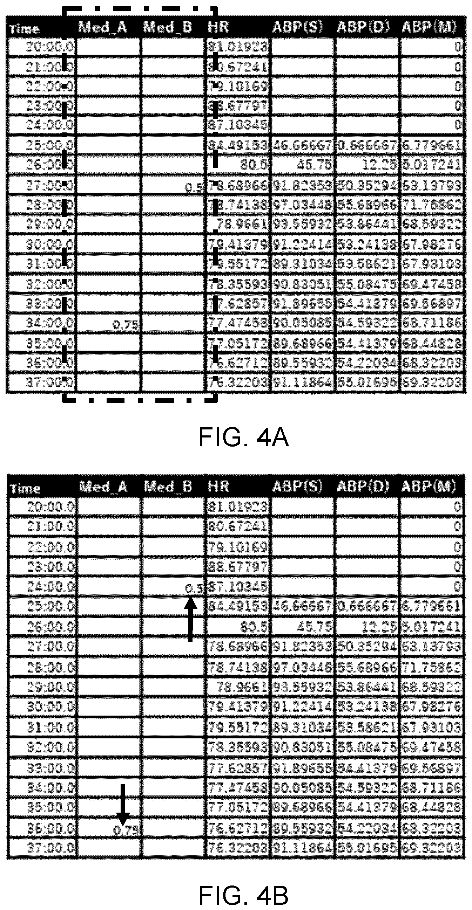

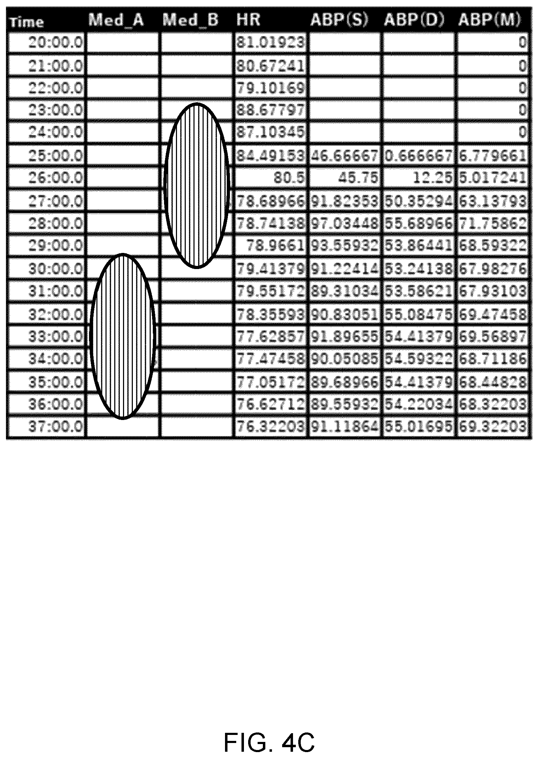

Moving onto Figure 4, there is provided a simplified block diagram of a system 400 for correcting machine-learning model training data according to an embodiment. The system 400 comprises an interface 410 and a data manipulation unit 420.

The interface 410 is configured to obtain training data comprising a timestamp value describing a timing of an event occurrence. The training data may be obtained from a database, or from another corpus of information.

The data manipulation unit 420 is configured to modify the timestamp value of the obtained training data according to a time-shift function configured to adjust the timestamp value based on a predetermined time uncertainty range. The functions of the data manipulation unit 420 and the interface 410 are described in more detail above, in reference to Figure 1. Indeed, this system 400 may be capable of performing the method 100 described in reference to Figure 1.

Thus, corrected training data may be acquired from the system 400, which may be used for more effective training of a machine-learning model.

In further embodiments, there may be provided a machine-learning model generation unit configured to generate a status prediction model adapted to output a status prediction indicative of a future physiological state of a subject, according to a method 200 described in reference to Figure 2. In other embodiments, there may also be provided a subject status prediction unit configured to generate a status prediction indicative of a future physiological state of a subject, according to a method 300 described in reference to Figure 3.

Figure 7 illustrates an example of a computer 1000 within which one or more parts of an embodiment may be employed. Various operations discussed above may utilize the capabilities of the computer 1000. For example, one or more parts of a system for correcting machine-learning model training data may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figures 1-3, and the systems described in relation to Figure 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and nonvolatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Figure 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A method (100) for correcting machine-learning model training data, the method comprising:

   obtaining (110) training data comprising a timestamp value describing a timing of an event occurrence; and
   modifying (120) the timestamp value of the obtained training data according to a time-shift function configured to adjust the timestamp value based on a predetermined time uncertainty range.

2. The method of claim 1, wherein the predetermined time uncertainty range is indicative of a predicted difference between the timestamp value and an actual timing of the event occurrence.

3. The method of claim 2, wherein the predetermined time uncertainty range is based on an event type corresponding to the event occurrence.

4. The method of any of claims 1-3, wherein the time-shift function is configured to adjust the timestamp value based on the predetermined time uncertainty range and a probability distribution algorithm.

5. The method of claim 4, wherein the probability distribution algorithm follows a uniform distribution.

6. The method of claim 4, wherein the probability distribution algorithm follows a normal distribution.

7. The method of claim 4, wherein the probability distribution algorithm follows an asymmetric probability distribution, and preferably a lognormal distribution.

8. A method (200) of generating a status prediction model adapted to output a status prediction indicative of a future physiological state of a subject, the method comprising:

> obtaining (210) time-series data comprising status data describing at least one physiological characteristic, and event data comprising a timestamp value describing a timing of an event occurrence;
> correcting (100) the event data according to any of claim 1-7; and
> training (220) a status prediction model using a training algorithm configured to receive an array of training inputs and known outputs, wherein the training inputs comprise the corrected event data and the status data, and the known outputs comprise the status data.

9. A method (200) of generating a status prediction model adapted to output a status prediction indicative of a future physiological state of a subject, the method comprising:

> obtaining (210) time-series data comprising status data describing at least one physiological characteristic, and event data comprising a timestamp value describing a timing of an event occurrence;
> correcting (100) the event data by modifying at least one of the timestamp values of the event data according to a time-shift function configured to adjust the timestamp value based on a predetermined time uncertainty range; and
> training (220) a status prediction model using a training algorithm configured to receive an array of training inputs and known outputs, wherein the training inputs comprise the corrected event data and the status data, and the known outputs comprise the status data.

10. The method of claim 9, wherein the status data comprises vital sign data, and preferably comprises at least one of a heart rate, a blood pressure, and an oxygen saturation level.

11. The method of claim 9 or 10, wherein the event data comprises intervention information describing a subject treatment, and preferably comprises at least one of a drug administration event, a movement event, and a treatment event.

12. The method of any of claims 9-11, wherein the training algorithm is a stochastic gradient descent algorithm.

13. A method (300) of generating a status prediction indicative of a future physiological state of a subject, the method comprising:

> generating (200) a status prediction model according to any of claims 8-11;
> obtaining (310) time-series data associated with the subject, the time-series data comprising status data describing at least one physiological characteristic of the subject, and event data comprising a timestamp value describing a timing of an event occurrence corresponding to the subject;
> acquiring (320) the subject status prediction based on inputting the time-series data to the generated status prediction model.

14. A computer program comprising computer program code means adapted, when said computer program is run on a computer, to implement the method of any of claims 1-13.

15. A system (400) for correcting machine-learning model training data, the system comprising:

> an interface (410) configured to obtain training data comprising a timestamp value describing a timing of an event occurrence; and
> a data manipulation unit (420) configured to modify the timestamp value of the obtained training data according to a time-shift function configured to adjust the timestamp value based on a predetermined time uncertainty range.

100

Obtain training data —110

Modify timestamp value of the training data —120

FIG. 1

200

Obtain time-series data and event data ——210

Correct the event data ——100

Train a status prediction model using the corrected event data and the status data ——220

FIG. 2

300

Generate a status prediction model ⌐200

Obtain time-series data ⌐310

Input the time-series data to the status prediction model ⌐320

Acquire subject status prediction ⌐330

FIG. 3

| Time | Med_A | Med_B | HR | ABP(S) | ABP(D) | ABP(M) |
|---|---|---|---|---|---|---|
| 20:00.0 | | | 81.01923 | | | 0 |
| 21:00.0 | | | 80.67241 | | | 0 |
| 22:00.0 | | | 79.10169 | | | 0 |
| 23:00.0 | | | 88.67797 | | | 0 |
| 24:00.0 | | | 87.10345 | | | 0 |
| 25:00.0 | | | 84.49153 | 46.66667 | 0.666667 | 6.779661 |
| 26:00.0 | | | 80.5 | 45.75 | 12.25 | 5.017241 |
| 27:00.0 | | 0.5 | 78.68966 | 91.82353 | 50.35294 | 63.13793 |
| 28:00.0 | | | 78.74138 | 97.03448 | 55.68966 | 71.75862 |
| 29:00.0 | | | 78.9661 | 93.55932 | 53.86441 | 68.59322 |
| 30:00.0 | | | 79.41379 | 91.22414 | 53.24138 | 67.98276 |
| 31:00.0 | | | 79.55172 | 89.31034 | 53.58621 | 67.93103 |
| 32:00.0 | | | 78.35593 | 90.83051 | 55.08475 | 69.47458 |
| 33:00.0 | | | 77.62857 | 91.89655 | 54.41379 | 69.56897 |
| 34:00.0 | 0.75 | | 77.47458 | 90.05085 | 54.59322 | 68.71186 |
| 35:00.0 | | | 77.05172 | 89.68966 | 54.41379 | 68.44828 |
| 36:00.0 | | | 76.62712 | 89.55932 | 54.22034 | 68.32203 |
| 37:00.0 | | | 76.32203 | 91.11864 | 55.01695 | 69.32203 |

FIG. 4A

| Time | Med_A | Med_B | HR | ABP(S) | ABP(D) | ABP(M) |
|---|---|---|---|---|---|---|
| 20:00.0 | | | 81.01923 | | | 0 |
| 21:00.0 | | | 80.67241 | | | 0 |
| 22:00.0 | | | 79.10169 | | | 0 |
| 23:00.0 | | | 88.67797 | | | 0 |
| 24:00.0 | | 0.5 | 87.10345 | | | 0 |
| 25:00.0 | | | 84.49153 | 46.66667 | 0.666667 | 6.779661 |
| 26:00.0 | | | 80.5 | 45.75 | 12.25 | 5.017241 |
| 27:00.0 | | | 78.68966 | 91.82353 | 50.35294 | 63.13793 |
| 28:00.0 | | | 78.74138 | 97.03448 | 55.68966 | 71.75862 |
| 29:00.0 | | | 78.9661 | 93.55932 | 53.86441 | 68.59322 |
| 30:00.0 | | | 79.41379 | 91.22414 | 53.24138 | 67.98276 |
| 31:00.0 | | | 79.55172 | 89.31034 | 53.58621 | 67.93103 |
| 32:00.0 | | | 78.35593 | 90.83051 | 55.08475 | 69.47458 |
| 33:00.0 | | | 77.62857 | 91.89655 | 54.41379 | 69.56897 |
| 34:00.0 | | | 77.47458 | 90.05085 | 54.59322 | 68.71186 |
| 35:00.0 | | | 77.05172 | 89.68966 | 54.41379 | 68.44828 |
| 36:00.0 | 0.75 | | 76.62712 | 89.55932 | 54.22034 | 68.32203 |
| 37:00.0 | | | 76.32203 | 91.11864 | 55.01695 | 69.32203 |

FIG. 4B

| Time | Med_A | Med_B | HR | ABP(S) | ABP(D) | ABP(M) |
|---|---|---|---|---|---|---|
| 20:00.0 | | | 81.01923 | | | 0 |
| 21:00.0 | | | 80.67241 | | | 0 |
| 22:00.0 | | | 79.10169 | | | 0 |
| 23:00.0 | | | 88.67797 | | | 0 |
| 24:00.0 | | | 87.10345 | | | 0 |
| 25:00.0 | | | 84.49153 | 46.66667 | 0.666667 | 6.779661 |
| 26:00.0 | | | 80.5 | 45.75 | 12.25 | 5.017241 |
| 27:00.0 | | | 78.68966 | 91.82353 | 50.35294 | 63.13793 |
| 28:00.0 | | | 78.74138 | 97.03448 | 55.68966 | 71.75862 |
| 29:00.0 | | | 78.9661 | 93.55932 | 53.86441 | 68.59322 |
| 30:00.0 | | | 79.41379 | 91.22414 | 53.24138 | 67.98276 |
| 31:00.0 | | | 79.55172 | 89.31034 | 53.58621 | 67.93103 |
| 32:00.0 | | | 78.35593 | 90.83051 | 55.08475 | 69.47458 |
| 33:00.0 | | | 77.62857 | 91.89655 | 54.41379 | 69.56897 |
| 34:00.0 | | | 77.47458 | 90.05085 | 54.59322 | 68.71186 |
| 35:00.0 | | | 77.05172 | 89.68966 | 54.41379 | 68.44828 |
| 36:00.0 | | | 76.62712 | 89.55932 | 54.22034 | 68.32203 |
| 37:00.0 | | | 76.32203 | 91.11864 | 55.01695 | 69.32203 |

FIG. 4C

400

Interface ~410

Data
Manipulation
Unit ~420

FIG. 5

FIG. 6

Europäisches
Patentamt

European
Patent Office

Office européen
des brevets

**EUROPEAN SEARCH REPORT**

Application Number

EP 22 18 2114

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/034590 A1 (OREN EYAL [US] ET AL) 31 January 2019 (2019-01-31) <br> * abstract * <br> * paragraph [0011] * <br> * paragraph [0022] * <br> * paragraph [0037] – paragraph [0040] * <br> * paragraph [0084] – paragraph [0086] * <br> * claims 1,7 * <br> * figure 2 * | 1-15 | INV. <br> G06N3/08 |
| X | LAURITSEN SIMON MEYER ET AL: "Explainable artificial intelligence model to predict acute critical illness from electronic health records", <br> NATURE COMMUNICATIONS, <br> vol. 11, no. 1, 31 July 2020 (2020-07-31), <br> XP093007391, <br> DOI: 10.1038/s41467-020-17431-x <br> Retrieved from the Internet: <br> URL:https://www.nature.com/articles/s41467 -020-17431-x> <br> * abstract * <br> * page 5, right-hand column, line 18 – page 6, right-hand column, line 9 * <br> * tables 1-3 * | 1-15 | |
| X | JENNA WONG ET AL: "Using Machine Learning to Identify Health Outcomes from Electronic Health Record Data", <br> CURRENT EPIDEMIOLOGY REPORTS, <br> vol. 5, no. 4, <br> 20 September 2018 (2018-09-20), pages 331-342, XP055690390, <br> DOI: 10.1007/s40471-018-0165-9 <br> * abstract * <br> * Section 2 * <br> * Section 3.1 * <br> * Section 3.2 * | 1-15 | TECHNICAL FIELDS SEARCHED (IPC) <br><br> G06N |

The present search report has been drawn up for all claims

| Place of search | Date of completion of the search | Examiner |
|---|---|---|
| Berlin | 13 December 2022 | Pose Rodríguez, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 2114

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2022

| Patent document cited in search report | | Publication date | Patent family member(s) | | Publication date |
|---|---|---|---|---|---|
| US 2019034590 A1 | | 31-01-2019 | CN | 110691548 A | 14-01-2020 |
| | | | EP | 3634204 A1 | 15-04-2020 |
| | | | JP | 7030853 B2 | 07-03-2022 |
| | | | JP | 2020529057 A | 01-10-2020 |
| | | | KR | 20200003407 A | 09-01-2020 |
| | | | US | 2019034589 A1 | 31-01-2019 |
| | | | US | 2019034590 A1 | 31-01-2019 |
| | | | US | 2019034591 A1 | 31-01-2019 |
| | | | WO | 2019022779 A1 | 31-01-2019 |

EPO FORM P0459

For more details about this annex : see Official Journal of the European Patent Office, No. 12/82